# EUROPEAN PATENT APPLICATION

(11) **EP 3 992 987 A1**
(43) Date of publication of application: **04.05.2022**
(21) Application number: 21205244.3
(22) Date of filing: 28.10.2021
(51) Int. Cl.: G16H 80/00, G06F 3/01, G06Q 50/00, G05D 1/00

(54) **SYSTEM AND METHOD FOR CONTINOUSLY SHARING BEHAVIORAL STATES OF A CREATURE**

(30) Priority: 30.10.2020 US 202017084646
(71) Applicant: Honda Research Institute Europe GmbH, 63073 Offenbach/Main (DE); Sproutel Inc., Providence, Rhode Island 02909 (US)
(72) Inventor: Weisswange, Thomas, 63073 Offenbach/Main (DE); Schmüdderich, Jens, 63073 Offenbach/Main (DE); Horowitz, Aaron, Providence, Rhode Island 02909 (US); Schwartz, Joel, Los Angeles, California 90064 (US)
(74) Representative: Beder, Jens

(57) **Abstract**

The present disclosure relates to a system for continuously sharing behavioral states of a creature. The system comprises a robot (12) configured to carry out different behaviors, at least one sensing means (5), (6), (8), (9) for automatically sensing the creature, a determining means for continuously determining the state of the creature based on information derived from the at least one sensing means (5), (6), (8), (9) and a selecting means for selecting, from the different behaviors, a behavior that is assigned to the determined state, wherein the robot (12) is configured to carry out the selected behavior.

## Description

The present disclosure relates to a technique for detecting activities and other states of a person and reporting these to a remote person. The disclosure, however, is not limited to persons, but can also be applied to other creatures like animals.

In elderly care, systems exist that detect negative states of a person living alone and report these to a caretaker or allow the person to communicate an emergency service. For example, a device that detects falls of a person and reports those to a caretaker is described in US 2009048540 A1. US 20090322513 A1 describes a method that measures physiological health parameters and reports emergency cases along with the location to a caretaker.

However, in such monitoring systems, a small number of defined states is detected and reported as singular events in real-time.

Further, telepresence systems designed for active communication and or providing a feeling of presence at a remote place through explicit interaction are known. US 9552056 B1 discloses a telepresence robot device that mirrors the gestures of the remote operator to communicate them to the receiver.

However, telepresence systems are not designed to communicate regular activities, particularly in a continuous way to remote persons.

Kwangmin Jeong et al.: "Fribo: A Social Networking Robot for Increasing Social Connectedness through Sharing Daily Home Activities from Living Noise Data", Proceedings of the 2018 ACM/IEEE International Conference on Human-Robot Interaction (2018) discloses a social robot that recognizes user's activity by analyzing occupants living noise and shares the activity information with close friends through their own robots. Particularly, this document discloses a system comprising sensors that are located within a house or apartment, and which generate sensor data of living noise (activity data), and a social robot that receives the sensor data in real-time, translates the sensor data into high-level information, and transmits this information to another robot of the same kind, which outputs the high-level information to a second user using a speaker and a display.

However, since the high-level information is output only as spoken or displayed information, the social robot stimulates only a few senses, requires explicit attention to the device and can only communicate events at a certain time and therefore hardly gives the impression of the continuous presence of a person.

Thus, it is an object of the present disclosure to improve the performance of a system and method for continuously sharing states of a creature.

The present disclosure provides a system and method for continuously sharing behavioral states of a creature.

The system according to the disclosure for continuously sharing behavioral states of a creature comprises a robot configured to continuously carry out different behaviors, at least one sensing means for automatically sensing a creature, a determining means for determining the state of the creature based on information derived from the at least one sensing means and a selecting means for selecting, from the different behaviors, a behavior that is assigned to the determined state, wherein the robot is configured to carry out the selected behavior. In order to continuously communicate the state, the same behavior can be repeated until a new (next) state of the creature is determined by the determining means. Determining the state of the creature is performed continuously.

The method according to the disclosure for continuously sharing behavioral states of a creature uses a robot configured to carry out different behaviors and comprises the steps of:
- automatically sensing a creature by at least one sensing means;
- continuously determining the state of the creature based on information derived from the at least one sensing means;
- selecting, from the different behaviors, a behavior that is assigned to the determined state; and
- carrying out the selected behavior by the robot.

The system according to the disclosure for continuously sharing behavioral states of a creature comprises a robot configured to continuously carry out different behaviors, at least one sensing means for automatically sensing a creature, a determining means for determining the state of the creature based on information derived from the at least one sensing means and a selecting means for selecting, from the different behaviors, a behavior that is assigned to the determined state, wherein the robot is configured to carry out the selected behavior. In order to continuously communicate the state, the same behavior can be repeated until a new (next) state of the creature is determined by the determining means. Determining the state of the creature is performed continuously.

With the present disclosure, the robot communicates through its behavior the abstracted status of a remote creature in real-time and through this enables a passive and continuous telepresence of this creature and reveals opportunities for shared activities and experiences. It is to be noted that the expression "real-time" shall also cover delays caused by signal processing and transmission. Since the robot communicates through its behavior, the robot stimulates many senses, requires no explicit attention and gives a good impression of the continuous physical presence of the creature. An improvement over the state of the art is that the system according to the present disclosure enables a continuous state communication, which goes beyond the event character of messages (i.e. systems that only communicate at a distinct point in time, while the robot according to the disclosure will at any time communicate something). This leads to the fact, that it is not necessary to focus attention on the robot at that particular moment in time ("active") but rather communication will also have an effect if the attention to the robot is happening at any other time. In any case, observing and communicating behavior of a remote creature improves the impression of the real companion.

The creature can be a person or a pet, wherein the robot communicates with another person(s) or pet(s), e.g. states of a pet are shared with a person, who is not permitted to keep pets.

In order to carry out the selected behavior, the robot can be configured to move to a predetermined position in the environment of the robot, to move along a predetermined trajectory, to move with a predetermined velocity profile, to perform a predetermined pose, to output a predetermined sound and/or to output a light in a predetermined color and/or in a predetermined intensity.

In addition, in order to carry out the different behaviors, the robot can be configured to move to different positions in the environment of the robot, to move on different trajectories, to move with different velocity profiles, to perform different poses, to output different sounds and/or to output light in different colors and/or different intensities.

The robot can operate in the environment of the user, e.g. on the floor of his apartment, or in a miniature model of an apartment or an animal cage. For example, the system can comprise a miniature model of the environment of the creature, wherein the robot is configured to move in the miniature model (e.g. small robot using at least in part, external sensors installed in the model).

The system can operate in a form of telepresence, in which the robot is at the home of one user or a user group (the "host" user/location), whereas the corresponding sensor system is located in the space of another user (the "remote" user/location). The creature and the robot are located in different places and the system can comprise a transmitting means for transmitting, via a data network, the information derived from the at least one sensing means located at the "remote" user/location to the determining means located at the "host" user/location, the determined state from the determining means located at the "remote" user/location to the selecting means located at the "host" user/location or the selected behavior from the selecting means located at the "remote" user/location to the robot located at the "host" user/location.

The detection means can use image processing methods to classify between a predefined number of state classes (e.g. a machine learning method trained with labeled videos of people demonstrating a certain state) or directly use sensors or signals from certain devices, e.g. TV communicating its power status, fridge communicating if its door is open, together with a set of predefined rules how to map these signals to the predefined states, i.e. "watching TV" or "eating".

The states determined by the determining means are:
- emotional states, like being happy, aroused, bored, relaxed, captivated, stressed;
- physiological states, like sleeping, being awake, exhausted, active, cold/hot;
- performed activities like cooking, watching TV, on the phone, reading, knitting;
- other specific cases, like being not at home, available for a call, idle; and/or
- emergency states, like fall, arrhythmia/stroke, illness.

For this, the determining means can be configured to determine a predetermined emotional state of the creature, a predetermined physiological state of the creature, a predetermined activity performed by the creature and/or a predetermined emergency state of the creature.

In addition, the determining means can be configured to classify the state of the creature into different emotional states, different physiological states, different activities and/or different emergency states.

To ensure the protection of privacy, the system can comprise a setting means for setting, by the "remote" or "host" user, states of the creature that are not allowed to share and/or times, in which states of the creature are not allowed to share. In this way, the remote user, or any operator configuring the system, can specify in a privacy setting, which states he is willing to share and/or he can actively activate/deactivate the sharing for some time.

For sensing the creature, static sensors, mobile sensors, (e.g. sensors in smart watches or smartphones) or signals indicating actuated devices (e.g. appliances of a smart home system) can be used. In another exemplary embodiment, at least one of the sensing means is configured to detect an operating state of a device controllable by the creature.

The following sensor types can be used:
- cameras, like 3D cameras, active ("pan-tilt-zoom") cameras, IR cameras;
- microphones
- physiological sensors, like skin conductance sensors, EMG, EEG, electrodes, optical heart beat sensors, temperature sensors;
- magnetic switch sensors, proximity sensors (e.g. Ultra sonic), motion sensors (e.g. optical), pressure sensors (e.g. piezo-electric); and/or
- virtual sensors, like for on-screen selection.

In another exemplary embodiment, at least one of the sensing means is a camera, a microphone, a physiological sensor, a proximity sensor, a motion sensor, a pressure sensor or a portable device configured to determine its location.

Alternatively or in addition, the system comprises at least one sensor robot equipped with at least one of the sensing means. The sensor robot can be configured to move in the environment of the creature and/or to direct the at least one of the sensing means to the creature.

In addition, the determining means can be configured to determine an interaction state, in which the creature interacts with the sensor robot at the remote site, wherein the selecting means is configured to select, when the interaction state is determined by the determining means, an interaction behavior, for example, that the robot moves to a predetermined object in the environment of the robot and/or circles around and/or interacts with the object. This interaction behavior can then be also triggered in the robot at the host location to create a mirrored impression and communicate the interaction state at the remote site.

The motion of the robot can be controlled based on one or more external and/or internal sensors of the robot. In another exemplary embodiment, the robot comprises at least one environment sensor (e.g. proximity sensor, camera) generating a signal indicative of the predetermined object, wherein the robot is configured to move to the predetermined object and/or to circle around and/or interact with the object based on the signal derived from the at least one environment sensor.

The method according to the disclosure for continuously sharing behavioral states of a creature uses a robot configured to carry out different behaviors and comprises the steps of:
- automatically sensing a creature by at least one sensing means;
- continuously determining the state of the creature based on information derived from the at least one sensing means;
- selecting, from the different behaviors, a behavior that is assigned to the determined state; and
- carrying out the selected behavior by the robot.

The disclosure will be described in the following in detail in particular with reference to the annexed drawings in which
- FIG. 1: shows a system according to an exemplary embodiment of the present disclosure;
- FIG. 2: shows a block diagram of a first part of the system shown in FIG. 1;
- FIG. 3: shows a block diagram of a second part of the system shown in FIG. 1; and
- FIG. 4: shows a simplified flow chart for explanation of the method for executing autonomously or partially autonomously driving according to the disclosure.

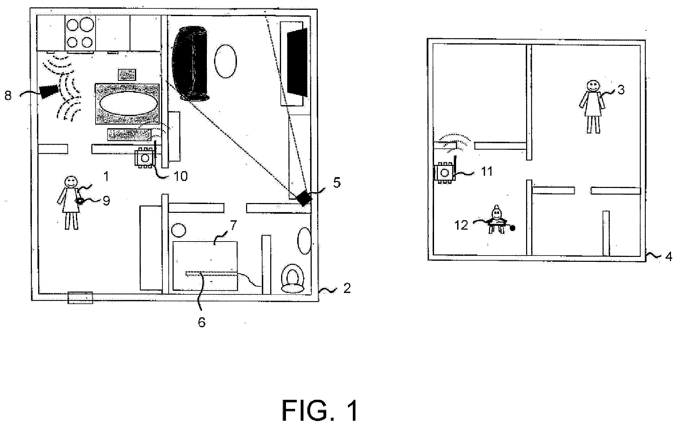

FIG. 1 shows a system according to an exemplary embodiment of the present disclosure, which shares behavioral states of a first person 1 ("remote" user) in a first apartment 2 with a second person 3 ("host" user) in a second apartment 4. The system shown in FIG. 1 comprises, in the first apartment 2, a camera 5 that captures an area in the living room, a pressure sensor 6 installed in a bed 7 in the bedroom, a microphone 8 installed in the kitchen, a smart watch 9 worn on the wrist of the first person 1 and a first processing unit 10 like a computer. In the second apartment 2, the system comprises a second processing unit 11, for example another computer, that is connected to the first processing unit 10 via a data network (not shown) and a robot 12 that communicates through its behavior the abstracted status of the first person 1.

The microphone 8 is configured to detect noise emerging from activities of the first person 1, like cooking, eating, walking, loud laughter and speaking and to transmit the detected noise to the first processing unit 10 via radio frequency communications or cables. The camera 5 generates an image signal from the first person 1 in the living room and transmits the image signal to the first processing unit 10 via radio frequency communication or cable. The pressure sensor 6 generates a pressure signal that indicates if the first person 1 is in the bed 7 or not and transmits the pressure signal to the first processing unit 10 via radio frequency communication or cable. The smart watch 9 comprises a plurality of sensors, like an ambient light sensor, a three-axis accelerometer, heart rate monitor and a positioning system (indoor and/or GPS) and is configured to transmit data generated by the sensors to the first processing unit 10 via radio frequency communication.

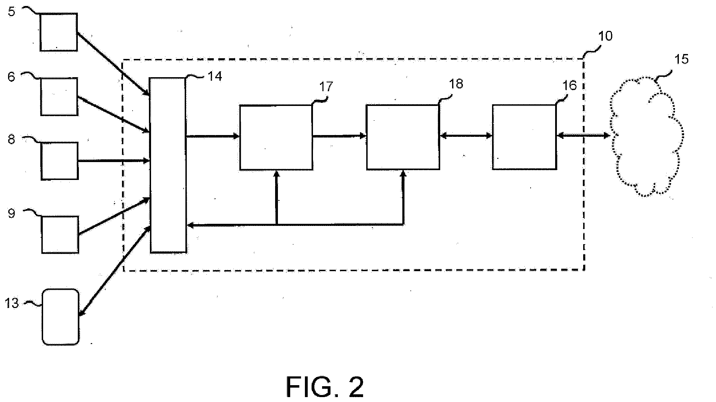

FIG. 2 shows a block diagram of the first processing unit 10 that is connected to the camera 5, the pressure sensor 6, the microphone 8, the smart watch 9 and an operating device 13 by a first transmitting and receiving means 14 (e.g. Bluetooth or WLAN transmission device) and that is connected to the data network 15 by a second transmitting and receiving means 16 (e.g. Internet router, residential gateway). The first processing unit 10 comprises a determining means 17 for determining the state of the first person 1 based on the sensor data received by the first transmitting and receiving means 14 and a setting means 18 for setting the states of the first person 1 that are not allowed to share and/or the times, in which states determined by the determining means are not allowed to share.

The determining means 17 determines continuously one or more states of the first person 1 based on the sensor data that are automatically generated by the camera 5, the pressure sensor 6, the microphone 8 and the smart watch 9, wherein the determining means 17 detects, in the sensor data, certain characteristics that are assigned to predetermined states.

In particular, the emotional state "happy" can be estimated by image and audio processing methods, wherein a smiling face is detected in the image captured by the camera 5 and a laughter is detected in the noise captured by the microphone 8, the physiological states "sleeping" and "awake" can be detected based on the pressure signal of the pressure sensor 6 and the heart rates measured by the smart watch 9, the states "watching TV", "on the phone", "reading" and "knitting" can be detected based on the image captured by the camera 5, the states "cooking" and "eating" can be detected based on the noise captured by the microphone 8, the emergency state "fall" can be detected based on the signal of the three-axis accelerometer of the smart watch 9 and the states "aroused", "captivated" and "stressed" can be estimated by speech recognition. Speech recognition per se is known from the prior art and also the use of the image and audio processing methods for detecting certain events.

The accuracy of the state determination could be improved by detecting a set of characteristics, wherein, for example, the state "away from home" can be set if the connection to the smart watch 9 fails, the pressure signal indicates "awake", the first person 1 is not captured by the camera 5 and noise or speech of the first person 1 is not detected for some time.

For each state (state class), one or more characteristics could be determined by a machine learning method trained with labeled noise and/or images (videos) of a person demonstrating the respective state (state class). The determining means 17 or an external device can perform the machine learning method, wherein the states and its corresponding characteristics or the learning data are inputted by the operating device 13 and stored in the detecting means 17.

Further, with the operating device 13, a user (e.g. the first person 1) can specify in a privacy setting, which states he is willing to share and/or he can actively activate/deactivate the sharing for some time. The setting is stored in the setting means 18, which generates a graphical user interface for the privacy setting and filters, from the states determined by determining means 17, states that are not allowed to share. The states allowed to share are received from the second transmitting and receiving means 16 that transmits the detected states to the second processing unit 11.

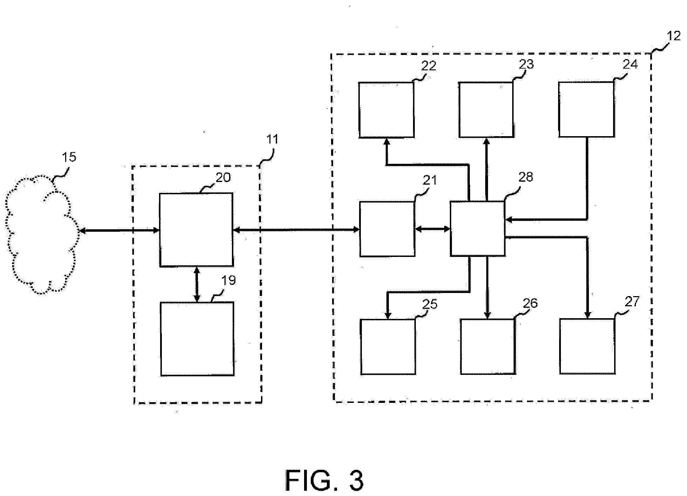

FIG. 3 shows a block diagram of the second processing unit 11 and the robot 12. The second processing unit 11 comprises a selecting means 19 that selects a behavior of the robot 12 based on the state(s) allowed to share and a transmitting and receiving means 20 (e.g. WLAN router) that is connected to the data network 15 to receive the state(s) from the first processing unit 10 and transmits the selected behavior to the robot 12. The robot 12 comprises a transmitting and receiving means 21 (e.g. WLAN adapter) that receives the selected behavior, a display 22 configured to display a symbolic representation of a face, a speaker 23 for outputting sounds, a camera 24 for sensing the environment of the robot 12, electrically driven legs 25, electrically driven arms 26, an electrically driven tail 27 and a controlling means 28 (e.g. microcontroller) that controls the display 22, the speaker 23, the electrically driven legs 25, the electrically driven arms 26 and the electrically driven tail 27 to perform the selected behavior.

All the means that are described in figures 2 and 3 may be realized as software modules that run on processors of the respective first processing unit 10, the second processing unit 11 or in the robot 12.

Example behaviors of the robot 12 could be:
- sitting in a specific position, either defined by an object, e.g. the second person 3, a dog basket, or a room-related location, e.g. kitchen, entrance door;
- facial expressions, e.g. smiling, yawning, closing eyes;
- gestures, e.g. waving; or body poses, e.g. lie down, stretch;
- general activities like walking around, jumping up&down, turning, dancing;
- specific activities like eating from a bowl, scratching a wall, playing with a ball;
- changing color, turning the display 22 on/off; and/or
- acoustic expressions like laughing, heavy breathing, snoring, purring.

The selecting means 19 stores to each state that can be determined by the determining means 17 a behavior, which represents an abstracted version of the respective state. In particular, if the first person 1 is awake and at home, a behavior, in which the eyes of the face displayed on the display 22 are open, is selected; if the first person 1 left the apartment 2, a behavior, in which the robot moves to a certain location, e.g. next to the entrance door and stays there, is selected; if the first person 1 is in a happy state, a behavior, in which the robot 12 smiles and/or wiggles its tail, is selected; and if the first person 1 is detected with activities that are interpreted as idle, a behavior, in which the robot 12 changes the color of the displayed face, is selected.

The steps of determining the state of the first person 1, selecting a behavior that is assigned to the determined state and communicating the state to the second person 3 by the selected behavior are automatically and continuously performed, without it being necessary to perform operating steps.

In a specific implementation, a symbolic representation of the robot 12 and its behavior can be displayed on a portable device (e.g. mobile phone or tablet having a companion app) so that the state can be shared, even if the second person 3 leaves the second apartment 4. Alternatively, the robot 12 or a part of the robot 12 is portable to show the behavior while being carried within or outside the second apartment 4, and/or the robot 12 is designed to operate in a miniature model of the second apartment 4 or another environment having multiple interaction points, like a doll's house, diorama or terrarium.

Alternatively or in addition, sensors for sensing the first person 1 could be installed within another (sensor) robot, similar or identical to the robot 12 located at the second apartment 4. In this case, interaction of the first person 1 with the sensor robot (looking, speaking and/or moving to the sensor robot) can be detected, which triggers a behavior in the sensor robot, and which is then "mirrored" to the robot 12.

Alternatively or in addition, for determining states, sensors or signals from certain devices, e.g. TV communicating its power status, fridge communicating if door is open, together with a set of predefined rules how to map these signals to assumed states, i.e. "watching TV" or "eating", can be used.

The functions of the determining means 17, the setting means 18 and/or the selecting means 19 described herein may be implemented using individual hardware circuitry, using software functioning in conjunction with a programmed microprocessor or a general purpose computer, using an application specific integrated circuit (ASIC) and/or using one or more digital signal processors (DSPs). Further, the determining means 17 or also the setting means 18 can be a part of the second processing unit 11, or the selecting means 19 can be a part of the first processing unit 10, wherein the transmitting and receiving means 20 receives the selected behavior and forwards the selected behavior to the robot 12.

Alternatively or in addition, the robot 12 can comprise a robotic arm with a fixed base position but multiple controllable joints; the robot 12 can be designed as legged "animal", which can move around freely and also express behaviors with its face or a wheeled robot with a screen.

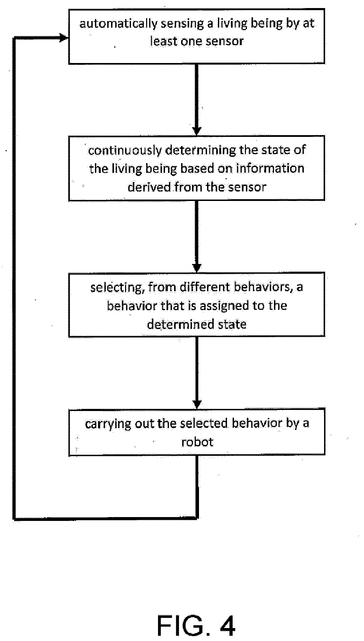

FIG. 4 shows a simplified flowchart showing the single steps performed by the realization of the method described in detail above.

It will be apparent to those skilled in the art that various modifications and variations can be made to the structure of the disclosure without departing from the scope or spirit of the disclosure. In view of the foregoing, it is intended that the present disclosure cover modifications and variations of this disclosure provided they fall within the scope of the following claims and their equivalents.

## Claims

1. A system for continuously sharing behavioral states of a creature, comprising
a robot (12) configured to carry out different behaviors;
at least one sensing means (5), (6), (8), (9) for sensing the creature;
a determining means (17) for determining the state of the creature based on information derived from the at least one sensing means (5), (6), (8), (9); and
a selecting means (19) for selecting, from the different behaviors, a behavior that is assigned to the determined state; wherein
the robot (12) is configured to carry out the selected behavior.

2. The system according to claim 1, wherein
the creature is a person or a pet.

3. The system according to claim 1, wherein
in order to carry out the selected behavior, the robot (12) is configured to move to a predetermined position in the environment of the robot (12), to move on a predetermined trajectory, to move with a predetermined velocity profile, to perform a predetermined pose, to output a predetermined sound and/or to output a light in a predetermined colour and/or in a predetermined intensity.

4. The system according to claim 3, wherein
in order to carry out the different behaviors, the robot (12) is configured to move to different positions in the environment of the robot (12), to move on different trajectories, to move with different velocity profiles, to perform different poses, to output different sounds and/or to output light in different colours and/or different intensities.

5. The system according to claim 1, further comprising
a miniature model of the environment of the creature; wherein
the robot (12) is configured to move in the miniature model.

6. The system according to claim 1, further comprising
the creature and the robot (12) are located in different places and the system further comprises a transmitting means (16) for transmitting, via data network (15), the information derived from the at least one sensing means (5), (6), (8), (9) to the determining means (17), the determined state to the selecting means (19) or the selected behavior to the robot.

7. The system according to claim 1, wherein
the determining means (17) is configured to determine a predetermined emotional state of the creature, a predetermined physiological state of the creature, a predetermined activity performed by the creature and/or a predetermined emergency state of the creature.

8. The system according to claim 7, wherein
the determining means (17) is configured to classify the state of the creature (1) into different emotional states, different physiological states, different activities and/or different emergency states.

9. The system according to claim 7, further comprising
a setting means (18) for setting, by a user, states of the creature that are not allowed to share and/or times, in which states of the creature are not allowed to share.

10. The system according to claim 1, wherein
at least one of the sensing means (5), (6), (8), (9) is configured to detect an operating state of a device controllable by the creature.

11. The system according to claim 1, wherein
at least one of the sensing means (5), (6), (8), (9) is a camera (5), a microphone (8), a physiological sensor, a proximity sensor, a motion sensor, a pressure sensor (6) or a portable device (9) configured to determine its location.

12. The system according to claim 1, further comprising
a sensor robot (12) comprising at least one of the sensing means (5), (6), (8), (9).

13. The system according to claim 12, wherein
the determining means (17) is configured to determine an interaction state, in which the creature interacts with the sensor robot (12); and
the selecting means (19) is configured to select, when the interaction state is determined by the determining means (17), an interaction behavior, in which the robot (12) moves to a predetermined object in the environment of the robot (12) and/or circles around the object.

14. The system according to claim 13, wherein
the robot (12) comprises at least one environment sensor generating a signal indicative of the predetermined object; and
the robot (12) is configured to move to the predetermined object and/or to circle around the object based on the signal derived from the at least one environment sensor.

15. A method for continuously sharing behavioral states of a creature using a robot configured to carry out different behaviors, the method comprising the steps of:
automatically sensing the creature by at least one sensing means (5), (6), (8), (9);
continuously determining the state of the creature based on information derived from the at least one sensing means (5), (6), (8), (9);
selecting, from the different behaviors, a behavior that is assigned to the determined state; and
carrying out the selected behavior by the robot (12).
